# EUROPEAN PATENT APPLICATION

(11) **EP 2 382 994 A1**
(43) Date of publication of application: **02.11.2011**
(21) Application number: 10161095.4
(22) Date of filing: 26.04.2010
(51) Int. Cl.: A61K 47/48

(54) **Ligand targeted nanocapsules for the delivery of RNAi and other agents**

(71) Applicant: Cattaneo, Maurizio Victor, Quincy, MA 02169 (US)
(72) Inventor: Cattaneo, Maurizio Victor, Quincy, MA 02169 (US)
(74) Representative: Colombet, Alain André

(57) **Abstract**

A carrier or carrier system for the delivery of therapeutic and/or diagnostic agents is described. The carrier system comprises ligands to target cells and enhance cell membrane penetration of therapeutic and/or diagnostic agents.

## Description

This invention relates to carriers, carrier systems and the delivery of therapeutic and/or diagnostic agents which are preferably targeted for site-specific release in cells, tissues and organs. In preferred embodiments, this invention relates to ligand-receptor mediated systems for target cell-specific delivery of nucleic acids, DNA, RNAi, oligonucleotides, proteins, peptides, drugs and/or diagnostic agents into cells.

The present invention relates to carriers for the delivery of therapeutic and/or diagnostic agents which are preferably targeted for site-specific release in cells, tissues or organs. More particularly, this invention relates to ligand-targeted polycation-coated liposomes which comprise a ligand and a biodegradable polycation for complexing polyanionic molecules such as nucleic acids and RNAi.

In spite of a substantial body of research and progress which has been achieved for the development of a system whereby a pharmaceutical agent can be selectively delivered to the site in need of treatment, many pharmaceutical delivery systems for the treatment of various diseases such as cancer, autoimmune, infectious and inflammatory diseases impart substantial risk to the patient.

Cancer continues to take a high toll on the American population (ACS. Facts and Figures. American Cancer Society. 2008) and an increasing number of biological and clinical studies in the last 20 years has been devoted not only to the discovery of mechanisms underlying such genetic disease, but mainly to the identification of specific targets for new molecular therapies. In fact, traditional systemic therapies such as chemo- and hormone-therapy have been showing a) considerable side effects, due to the susceptibility of normal cells to chemotherapy insults and b) failure in killing all cell populations in the context of the tumour, due to the fact that a portion of tumour cells is able to activate generic mechanisms of resistance to chemotherapeutic agents or hormones upon treatment. Also, the ultimate goal would be the realization of a molecular classification of all human cancers, based on which individual tumours could be treated independently of their origin, simply identifying the main alterations specifically responsible for tumour growth.

Although it is desired to concentrate a cytotoxic agent at a targeted site, current cancer treatment protocols for administering these cytotoxic agents typically call for repeated intravenous dosing, with careful monitoring of the patient. The dose is selected to be just below the amount that will produce acute (and sometimes chronic) toxicity that can lead to life-threatening cardiomyopathy, myelotoxicity, hepatic toxicity, or renal toxicity.

Previous attempts to administer such drugs by direct injection into the location of the organ having the malignancy are only partially effective, because of migration of the drug from that location and as a result of extensive tissue necrosis from extravasation. Such dispersion cannot be totally prevented, with the result that excessive quantities of drug need to be administered to attain a desired result.

The direct injection of cytotoxic agents into solid tumours of the breast, bladder, prostate and lung using conventional cytotoxic chemotherapeutic agents as adjuvants to surgery and/or radiotherapy has had limited success in prolonging the lives of patients. This is partially due to the dose limitations imposed by the acute and chronic toxicity to tissues or organ systems beyond those that are targeted.

For a compound to be an effective pharmaceutical agent *in vivo*, the compound must be readily deliverable to the patient, not rapidly cleared from the body, have a tolerable level of toxicity, and be able to reach the site within the body where it is needed.

In recent years there has been a strong focus on discovering new ways of targeting cancer cells. A major limitation to current cancer therapies is that they harm many healthy cells in the process.

The general problem of drug targeting consists of at least three basic issues. They are the following:
1. how to ensure the most effective interaction of drugs with target cells, including their proper binding on cell membranes and intracellular transport;
2. how to effectively deliver drugs towards certain target cells avoiding unfavourable drug distribution in the organism and their disintegration on their way to the targets;
3. how to avoid nonspecific action of drugs on nontarget cells.

Viral vectors are very effective in terms of transfection efficiency but they have limitations *in vivo* such as immunogenicity and unintended recombination (Douglas JT and Curiel DT. Targeted gene therapy. Tumour Targeting 1:67-84, 1995; Thomas CE, Ehrhardt A, Kay MA. Progress and problems with the use of viral vectors for gene therapy. Nat Rev Genet 4, 346-358, 2003; Verma IM and Somia N. Gene-therapy -promises, problems and prospects. Nat Med 389:239-242, 1997).

Non-viral delivery systems include cationic liposomes and cationic polymers. Cationic constructs are an attractive choice since simple mixing with negatively charged DNA or RNA *in vitro* leads to electrostatically-driven self-assembly into polyelectrolyte complexes (Kabanov AV and Alakhov VY. New approaches to targeting bioactive compounds. J Cont Release 28:15-35, 1994).

Liposomes are artificial single, oligo- or multi-lamellar vesicles having an aqueous core and being formed from amphipathic molecules. The cargo may be trapped in the core of the liposome or disposed in the membrane layer or at the membrane surface. Today, liposomal vectors are the most important group of the nonviral delivery systems. More specifically, cationic liposomes or lipids have been used widely in animal trials and/or clinical studies. Cationic liposomes have being used to deliver oligonucleotides and siRNA (Semple SC, Klimuk; Sandra K, Harasym T, Hope MJ, Ansell SM, Cullis P, Scherrer P, Debeyer Dan. Lipid-encapsulated polyanionic nucleic acid, U.S. Patent No. 6,858,225, 2005; Wheeler, JJ, Bally MB, Zhang YP, Reimer DL, Hope M, Cullis PR, Scherrer P. Lipid-nucleic acid particles prepared via a hydrophobic lipid-nucleic acid complex intermediate and use for gene transfer, U.S. Patent 5,976,567, 1999; Wheeler, JJ, Hope M, Cullis PR, Bally MB. Methods for encapsulating plasmids in lipid bilayers U.S. Patent 6,815,432, 2004). Also, a great deal of effort has been made over the years to develop liposomes that have targeting vectors such as monoclonal antibodies (mAbs) attached to the bilayer 4 surface (Barbet J, Machy P and Leserman LO. Monoclonal antibody covalently coupled to liposomes: specific targeting to cells. J. Supramolec Struct Cell Biochem 16:243-258, 1993; Jones MN and Hudson MJ. The targeting of immunoliposomes to tumour cells (A431) and the effects of encapsulated methotrexate. Biochim Biophys Acta 1152:231-242, 1993; Torchilin VP. Immobilization of specific proteins on liposome surface: systems for drug targeting. In: Liposome Technology vol.3, CRC, Boca Raton, FL, pp.29-40, 1993).

Although cationic systems provide high loading efficiencies, they lack colloidal stability, in particular after contact with body fluids. Ionic interactions with proteins and/or other biopolymers lead to *in situ* aggregate formation with the extracellular matrix or with cell surfaces. In addition, cationic liposomes, although they provide effective synthetic transfection systems, their use *in vivo* is limited by general toxicity, complement activation and liver and lung tropism (Dass CR. J. Pharm. Pharmacol 54:593-601, 2002; Dass CR. Lipoplex-mediated delivery of nucleic acids: factors affecting in vivo transfection. J Mol Med 82:579-91, 2004; Filion MC, Phillips NC. Toxicity and immunomodulatory activity of liposomal vectors formulated with cationic lipids toward immune effector cells. Biochim Biophys Acta 1329:345-356, 1997; Hirko A, Tang F, Hughes JA. Cationic lipid vectors for plasmid delivery. Curr. Med. Chem 10:1185-1193, 2003; Lv H, Zhang S, Wang B, Cui S, Yan J. Toxicity of cationic lipids and cationic polymers in gene delivery. J Cont Release 114:100-9, 2006; Ma Z, Li J, He F, Wilson A, Pitt B, Li S. Cationic lipids enhance siRNA-mediated interferon response in mice. Biochem Biophys Res Comm 330:755-9, 2005; Romoren K, Thu BJ, Bols NC, Evensen O. Transfection efficiency and cytotoxicity of cationic liposomes in salmonid cell lines of hepatocyte and macrophage origin. Biochim Biophys Acta 1663:127-34, 2004; Zhang J-S. Liu F, Huang L. Implications of pharmacokinetic behavior of lipoplex for its inflammatory toxicity. Adv Drug Del Rev 57:689-98, 2005). In addition, it has been shown that antibodies become immunogenic when coupled to these liposomes (Phillips NC and Dahman J. Immunogenicity of immunoliposomes: reactivity against species-specific IgG and liposomal phospholipids. Immunol Lett 45:149-52, 1995).

Cationic polymers have also frequently been selected for use as non-viral vectors. Studies with linear polycations such as poly(lysine) and a ligand such as a receptor-recognizing molecule do mimic some basic functions of natural viruses (Kabanov AV, Yu V, Alakhov VY, Chekhonin VP. Enhancement of macromolecular penetration into cells and nontraditional drug delivery systems, In: Skulachev VP (Ed.) Sov. Sci. Rev., D., Physicochem. Biol., Harwood Academic Publishers, New 5 York, Vol. 11, part 2, pp.1-75, 1992, Kabanov AV and Kabanov VA. DNA Complexes with Polycations for the Delivery of Genetic Material into Cells. Bioconj Chem 6:7-20, 1995). On the basis of this work Trubetskoy et al. (Trubetskoy VS, Torchilin VP, Kennel SJ and Huang L. Use of Nterminal modified poly(L-lysine)-antibody conjugate as a carrier for targeted gene delivery in mouse lung endothelial cells. Bioconjugate Chem 3:323-327, 1992) developed a system using poly (L-lysine) antibody conjugate as a carrier for targeted gene delivery in mouse lung endothelial cells. However, the cationic polymers most often used, including poly(lysine), polyethyleneimine and PAMAM dendrimers (Wu GY, Wu CH. Evidence for targeted gene delivery to HEP G2 hepatoma cells in vitro. Biochem 27:887-892, 1998) are very toxic to cells. Although the process of complexation with DNA or RNA, with the consequent charge neutralization counteracts this toxicity, it is nonetheless a concern when one considers the ultimate fate of the construct and the possibility for localized delivery of the polycation, hence the need for non-toxic polycations. Therefore, the toxicity of cationic lipids and polymers is still an obstacle to the application of non-viral vectors to gene therapy.

Recent data shows that a natural cationic biopolymer consisting of a low molecular weight highly purified chitosan was neither toxic nor hemolytic and could be administered intravenously without liver accumulation (Cattaneo MV and Demierre MF. Biodegradable Chitosan for Topical Delivery of Retinoic Acid. Drug Del Tech 1:44-48, 2001; Richardson SCW, Kolbe HVJ and R Duncan. Potential of low molecular mass chitosan as a DNA delivery system: biocompatibility, body distribution and ability to complex and protect DNA. Int J Pharm 178:231-243, 1999). The lactate form of this biocompatible polymer also showed rapid blood clearance and excellent DNA complexation resulting in inhibition of DNA degradation by DNase II, and greater DNA interaction than poly-L-lysine (Richardson SCW, Kolbe HVJ and Duncan R, 1999, Weecharangsan W. Opanasopit P. Ngawhirunpat T,. Rojanarata T, Apirakaramwong A. Chitosan lactate as a nonviral gene delivery vector in COS-1 cells. AAPS Pharm Sci Tech 7:66, 2006). Several investigators have included a moiety such as PEG to increase stealth and the circulation time of the drug carrier. PEG micelles and liposomes have been prepared according to a method described in Zalipsky et al. (Polyethylene glycol chemistry, Biotechnical and Biomedical Applications (J.M. Harris Ed.) Plenum Press, pp.347-370, 1992). In addition, since chitosan shows high affinity for lipids, several investigators have utilized chitosan derivatives as coating materials for liposomes (Guo J, Ping Q, Jiang G, Huang L and Tong Y. Chitosan-coated liposomes: characterization and interaction with leoprolide. Int J Pharm 260:167-173, 2003; Janes KA, Calvo P, Alonso MJ. Polysaccharide colloidal particles as delivery systems for macromolecules. Adv Drug Deliv Rev 47:83-97, 2001; Takeuchi H., Yamamoto H. and Kawashima Y. Mucoadhesive nanoparticulate systems for peptide drug delivery. Adv Drug Deliv Rev 47:39-54, 2001).

Transmembrane transport of nutrient molecules is a critical cellular function. Because practitioners have recognized the importance of transmembrane transport to many areas of medical and biological science, including drug therapy, peptide therapy and gene transfer, there have been significant research efforts directed to the understanding and application of such processes. Thus, for example, transmembrane delivery of nucleic acids has been encouraged through the use of protein carriers, antibody carriers, liposomal delivery systems, electroporation, direct injection, cell fusion, vital carriers, osmotic shock, and calcium-phosphate mediated transformation. However, many of those techniques are limited both by the types of cells in which transmembrane transport is enabled and by the conditions of use for successful transmembrane transport of exogenous molecular species. Further, many of these known techniques are limited in the type and size of exogenous molecule that can be transported across a membrane without loss of bioactivity.

One method for transmembrane delivery of exogenous molecules having a wide applicability is based on the mechanism of receptor-mediated endocytotic activity (REA). Unlike many other methods, REA can be used successfully both *in vivo* and *in vitro*. REA involves the movement of ligands bound to membrane receptors into the interior of an area bounded by the membrane through invagination of the membrane. The process is initiated or activated by the binding of a receptor-specific ligand to the receptor. Many REA systems have been characterized, including those recognizing peptide growth factors such as epidermal growth factor (EGF) and insulin growth factor, (IGF), galactose, mannose, mannose 6-phosphate, transferrin, asialoglycoprotein, transcobalamin (Vitamin B.sub.12), alpha-2-macroglobulins, insulin.
REA has been utilized for delivering exogenous molecules such as proteins and nucleic acids to cells. Generally, a specific ligand is chemically conjugated by covalent, ionic or hydrogen bonding to an exogenous molecule of interest (i.e. the exogenous compound), forming a conjugate molecule having a moiety (the ligand portion) that is still recognized in the conjugate by a target receptor. Using this technique the hepatocyte-specific receptor for galactose terminal asialoglycoproteins has been utilized for the hepatocyte-specific transmembrane delivery of asialoorosomucoid-poly-L-lysine non-covalently complexed to a DNA plasmid (Wu, G. Y. J. Biol Chem. 262:4429-4432, 1987); the cell receptor for epidermal growth factor has been utilized to deliver polynucleotides covalently linked to EGF to the cell interior (Myers AE, A method for internalizing nucleic acids into eukaryotic cells; European Patent Application No. 86810614, 1988).

With respect to RNAi delivery, a major limitation to the use of RNAi *in vivo* is the effective delivery of RNAi to the target cells (Behlke MA. Progress towards in vivo use of siRNAs. Mol Ther 13:644-70, 2006; Dykxhoorn DM, Lieberman J. Knocking down disease with siRNAs. Cell 126:231- 5, 2006). As a general rule, a molecule such as RNAi faces not one but a combination of problems. Although RNAi is a potentially useful therapeutic approach to silence the targeted gene of a particular disease, its use is limited by its stability *in vivo*. In particular, RNAi faces the problem of penetration into cells while avoiding disintegration in body fluids and intracellularly. Therefore, despite some progress achieved in this field, no reliable tool for siRNA targeting has yet been developed.
RNAi that specifically interferes with gene expression at the transcriptional or translational levels have the potential to be used as therapeutic agents to control the synthesis of deleterious proteins associated with viral, neoplastic or other diseases. These treatment strategies have been shown to block the expression of a gene or to produce a needed protein in cell culture. However, a major problem with these promising treatments, is adapting them for use *in vivo*.

Recently, there has also been an important focus on the application of RNAi to silence specific genes involved in cancer proliferation. Although RNAi has shown great efficacy in the selective inhibition of gene expression, the therapeutic applications of RNAi is currently limited by their low physiological stability, slow cellular uptake, and lack of tissue specificity.

Thus, there exists a need for a drug delivery system which can be utilized for the delivery of RNAi, which may also include therapeutic and/or diagnostic agents. There is also a need for a drug delivery system which can be used for site-specific release of RNAi, therapeutic and/or diagnostic agent in the cells, tissues, or organs in which a therapeutical effect is desired to be affected.

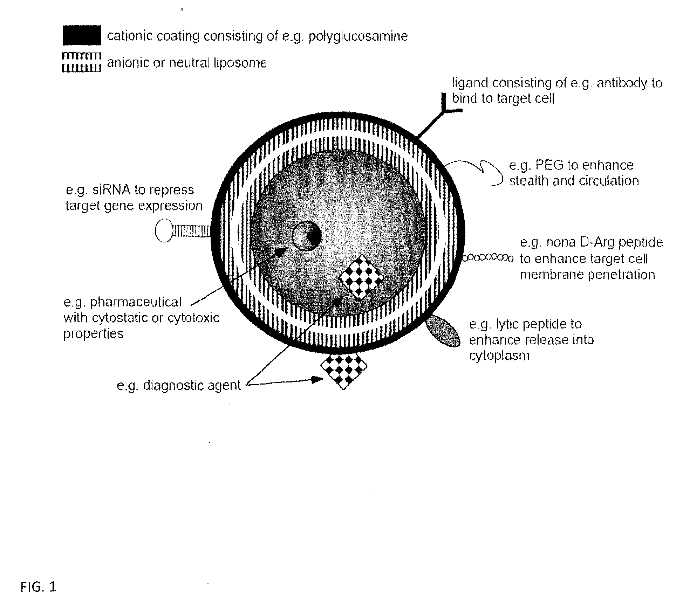

The present invention relates to carriers for the delivery of therapeutic and/or diagnostic agents which are preferably targeted for site-specific release in cells, tissues or organs. More particularly, this invention relates to ligand-targeted nanocapsules which comprise a ligand and a nanocapsule containing a polycation for complexing polyanionic molecules such as nucleic acid and RNA. Different coatings, ligands, RNAi, pharmaceuticals and/or diagnostics can be used tailored (customized) to the intended target cell in order to achieve maximum antitumour activity of the system as shown in Figure 1.

According to the present invention there is provided a carrier system, useful for administering therapeutic molecules to target cells, comprising ligand-targeted liposomes including on their surface a polymer chemically coupled to a ligand, said ligand
- possessing a high affinity for predefined receptor sites of the target cells;
- being capable of acting as a cell targeting agent of the target cells;
- being capable of acting as a cell internalization vector.

In particular, a carrier system according to the invention may comprise one or more of the features selected in the group consisting of
(a) the liposome is neutral or anionic and its vesicle-forming lipid is selected in the group consisting of hydrogenated soy phosphatidylcholine, cholesterol, DSPE-polyethyleneglycol-maleimide, distearoylphosphatidylcholine, sphingomyelin, diacyl glycerol, phosphatidyl ethanolamine, phosphatidylglycerol, distearylphosphatidylcholine and distearyl phosphatidylethanolamine ; and having a diameter of less than 2 microns, preferably less than 1 micron, more preferably having a diameter ranging from 300 to 500 nanometers, for example around 100 nanometers; and preferably comprising at least one polyethyleneglycol vesicle-forming lipid having a molecular weight of less than 10,000 Dalton or less than 5,000 Dalton or less than 3,400 Dalton and said lipid being able to improve stealth and circulatory properties of the system;
(b) the polymer is selected in the group consisting of a polysaccharide, polyglucosamine, oligoglucosamine, chitosan, a polypeptide, polyethylene glycol, polylysine, polyarginine and copolymers thereof ; and may contain an improving agent that is selected in the list consisting of the polyarginine D- and L-forms, nona-D-arginine, polylysine D- and L-forms, polyglucosamine and polyacetylglucosamine or mixtures thereof, and that is acting on the crossing, fusion or uptake of the carrier system across the target cell membrane;
(c) the ligand is an antibody Fab or a fragment thereof.

The carrier system according to the invention may also include a spacer, between the polymer and the improving agent, the spacer being preferably selected in the list consisting of polysaccharide, polyethylene glycol, poly(alkylene glycol), for example having a molecular weight less than 10,000 Dalton or in the list consisting of polysaccharide, polyethylene glycol, poly(alkylene glycol), for example having a molecular weight less 5000 Dalton or in the list consisting of polysaccharide, polyethylene glycol, poly(alkylene glycol), for example having a molecular weight less than or equal to 3400 Dalton.

The carrier system according to the invention may also include a lysis agent coupled to the polymer, in particular a pH-sensitive endosomolytic peptide lysis agent.

The carrier system according to the invention may also comprise a second ligand selected in the group consisting of an antigen, a hapten, a vitamin, a protein, a polypeptide, biotin, nucleic acids, DNA, RNA, aptamers, polynucleic acids, a polysaccharide, a carbohydrate, a lectin, a lipid and combination thereof and, preferably being
(i) connected to either the DSPE-polyethyleneglycol-maleimide lipid
   - in particular, by using a coupling agent such as 2-iminothiolane hydrochloride to convert the available amino groups into thiol groups for coupling to the maleimide group present on the pegylated lipid, or
   - by first using tris(2-carboxyethyl)phosphine to reduce the available sulfide linkages into sulfidryl groups; or
(ii) connected to N-(3-Dimethylaminopropyl)-N'-ethylcarbodiimide hydrochloride to couple free available amino or carboxyl groups on the polysaccharide coating (eg. chitosan, hyaluronic acid) to free carboxyl or amino groups on the ligand.
Preferably, the second ligand is able to bind to a viral antigen, the extracellular domain of signalling membrane proteins such as epidermal growth factor receptor, HER2/neu receptor, basic fibroblast growth factor receptor, vascular endothelial growth factor receptor, tumor necrosis factor receptor, insulin growth factor receptor, folate receptor, cell adhesion molecules such as E-selectin receptor, P-selectin receptor, L-selectin receptor, integrin receptors, chemokine receptors or other growth factor receptors, in particular the second ligand is able to bind to a target cell or tissue specific antigen, preferably to bind to a target cell or tissue specific antigen selected in the list consisting of prostate specific membrane antigen, TROY, lymphocyte antigens and tumour antigens.

The carrier system according to the invention is particularly useful for administering therapeutic molecules to internalizable target cells or antigen.

The combination of a low-toxicity biodegradable polycation with anionic or neutral liposomes, said polycation being coupled to target-specific ligands, produces a carrier system with a low potential systemic toxicity. The polycationic coating makes this system exquisitely suitable for coupling polyanionic agents such as siRNA. In addition, the liposomal component of the carrier system can be used to entrap therapeutic and/or diagnostic agents. Further modifications of the coating by molecules such as polyethylene glycol (PEG) can be implemented to further increase the blood circulation time. The polycation coating serves as a platform for both complexing the polyanions as well as covalently binding of ligands that increase the identification and subsequent penetration of the target cell membrane.

The cationic biopolymer acts as a transfecting agent and a carrier for anionic macromolecules as well as a matrix for coupling of different ligands results in a dramatic (at least 2 log order) increase in the transfection (delivery) efficiency of the nanocapsules compared to an antibody control particle (normal IgG). Furthermore, the liposomal component acts as a carrier for other therapeutic and/or diagnostic agents, thus separating the polyanionic agents such as RNAi from other therapeutic or diagnostic agents that are entrapped in the liposome. This would allow simultaneous delivery of agents that can interfere with two different biochemical pathways in the target cell.

This invention reveals that transfection efficiency without the specific antibody is negligible. Thus, it is conceivable that our ligand-coupled nanocapsules will achieve high tumour-specific delivery and reduce toxicity.

Ligand-targeted nanoparticles are interesting vectors since they may help protect the encapsulated drug from *in vivo* degradation as well as minimize the drug's toxicity as a result of the targeting feature of the molecular entity. In this context, the term "ligand" refers to a biomolecule which can bind to a specific receptor protein located on the surface of the target cell or in its nucleus or cytosol. The ligand is internalized through a process termed receptor-mediated endocytotic activity, where the receptor binds the ligand, the surrounding membrane closes off from the cell surface, and the internalized material then passes through the vesicular membrane into the cytoplasm. The ligand then becomes the transfecting agent.

In one embodiment, the ligand may be an antibody, hormone, pheromone, or neurotransmitter, or any biomolecule capable of acting like a ligand, which binds to the receptor. When the ligand binds to a particular cell receptor, endocytosis is stimulated. Examples of ligands which have been used with various cell types to enhance biomolecule transport are galactose, transferrin, the glycoprotein asialoorosomucoid, epidermal growth factor, fibroblast growth factor and folic acid.

If the ligand is chemically coupled to a carrier which contains or is complexed to an anionic macromolecule, the macromolecule can then enter the cytoplasm. The carrier can be a cationic polymer which will further enhance cell membrane penetration as well as complexing an anionic molecule such as RNAi.

Each carrier system may complex RNAi as well as carry a therapeutic and/or diagnostic agent by entrapment into the liposomal compartment of the carrier.

According to the present invention, the drawings represent:
FIG. 1A schematic representation of the carrier system;
FIG. 2
   (A) Antibody-coupled nanocapsule transfection efficiency to melanoma cells. The quantities used for this experiment were 2, 12.5, 25, 50, and 100 mg;
   (B) The quantities of antibodycoupled nanocapsules used for this experiment were 10, 15, 20, 25, and 30 mg;

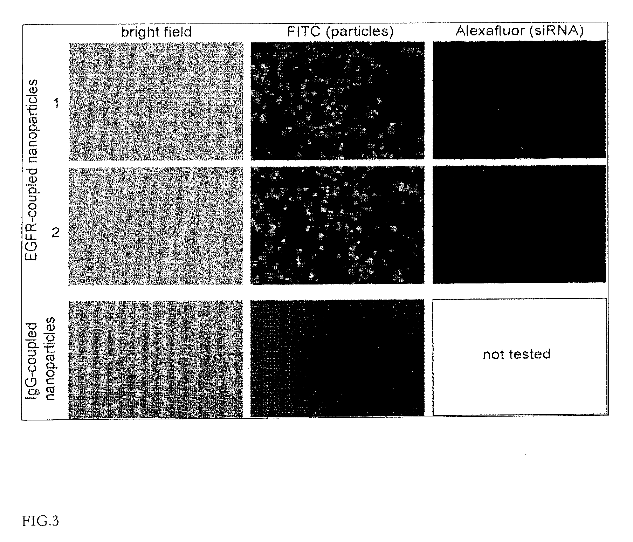

FIG. 3 Nanocapsule transfection efficiency to melanoma cells. The quantities used for this experiment were 20 mg of antibody-coupled nanocapsules containing 3' Alexa Fluor 488-labeled validated BRAF siRNA. The experiment was repeated twice for the EGFR-mAb nanocapsules. EGFR coupling to A375 melanoma cells is shown in green (FITC label) and siRNA incorporation in A375 melanoma cells is shown in red (Alexa Fluor 488 label). No FITC fluorescence is detectable with the IgG-pAb nanocapsules containing FITC label.;

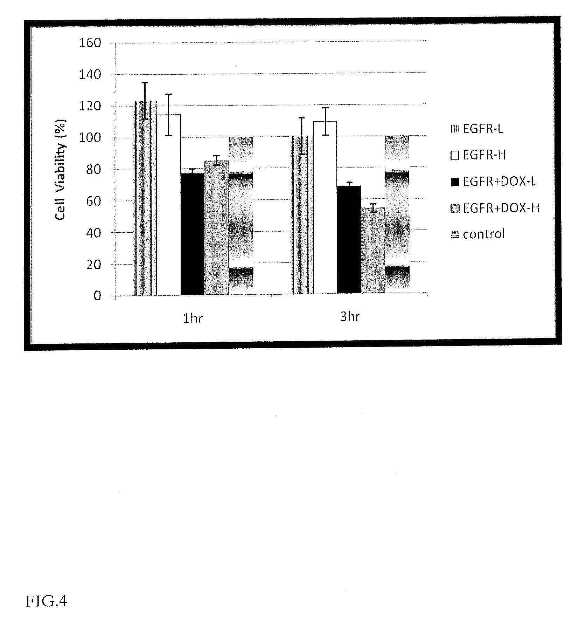

FIG. 4. MTS assay to determine cell viability and proliferation of DOX-encapsulated EGFRmAb at 2 different dosing (EGFR-H = 1.6 ml, and EFGR-L=2.5 ml,) without and with DOX (EGFR + DOX-L and EGFR-DOX-H) after incubation with A375 melanoma cells. The cells were incubated with nanocapsules for 1 hour (Left) and 3 hours (*Right*), and the cells were removed and incubated in fresh media for a total of 48 hours before assessing cell viability and proliferation. (Error bars represent ±1 % standard deviations from the mean).

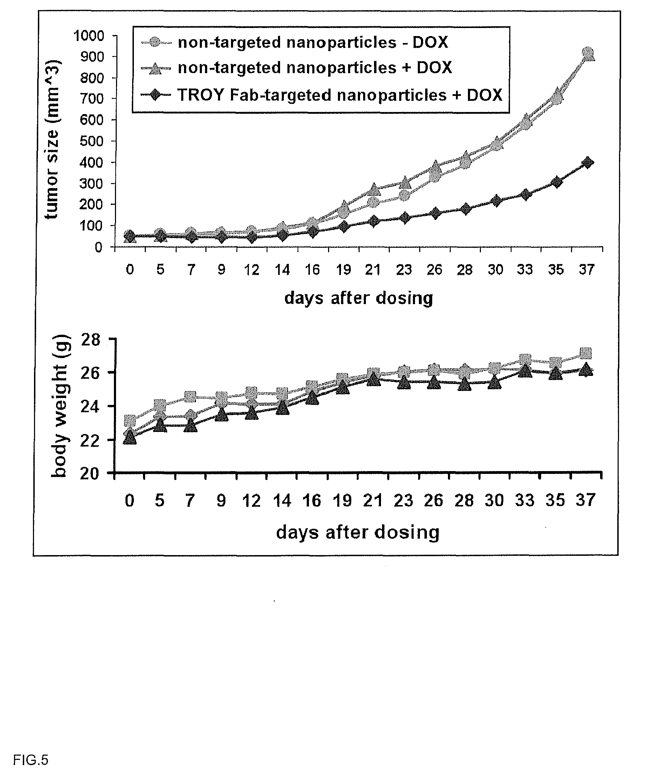

FIG. 5. Nude mouse experiments showing that TROY-targeted DOX-loaded immunoliposomes significantly inhibit A375 melanoma xenograft growth compared to non-targeted controls -/+ DOX. Variability was <10%. One way Anova, p<0.0309 and 0.0279, resp. There were no statistical differences in body weight, indicative of low toxicity.

The present invention relates to carriers for the delivery of therapeutics and/or diagnostic agents which are preferably targeted for site-specific release in cells, tissues or organs. More particularly, this invention relates to ligand-targeted nanocapsules consisting of biodegradable polycation coated liposomes, said polycation intended for complexing polyanionic molecules such as nucleic acid and RNAi and a ligand covalently attached to the polycation for triggering receptor mediated endocytosis.

The invention includes, in one embodiment, a nanocapsule including a coating of a hydrophilic cationic polymer on a liposome surface as shown in Figure 1, the cationic polymer serving to complex polyanionic macromolecules for transport in the circulation and across a membrane of a cell, and the liposome serving to prevent any aggregate formation generally occurring between the cationic polymer coating and the polyanionic macromolecule.

This invention includes in another embodiment the molecular weight of the polycationic polymer which is to be selected for optimal delivery of the polyanionic macromolecule to the target cell. The molecular weight can be in the range from about 100 to about 20,000. Preferably, the molecular weight of the polycationic polymer can be in the range from about 200 to about 10,000. Most preferably, a polycationic polymer with a molecular weight in the range from about 400 to about 2,000 can be used to deliver the polyanionic macromolecule to the cell.

The coating on the liposomal particle may consist of low molecular weight chitosan. The term chitosan refers to a family of polymers having a high percentage of glucosamine (normally 80%- 99%) and N-acetylated glucosamine (1%-20%). Low molecular weight chitosan forms a linear polysaccharide chain of molecular weight up to 20,000 Dalton. Chitosan is derived from chitin. It is normally extracted from the exoskeleton of shellfish, mushrooms, or algae and has been previously been described having controlled release properties. Highly purified chitosan, as obtained commercially under the tradename Protasan™ (Novamatrix™, FMC Biopolymers, Philadelphia, PA) is both biocompatible as well as biodegradable.

As used herein biocompatible refers to a substance that has limited immunogenic and allergenic ability. Biocompatible also means that the substance does not cause significant undesired physiological reactions. A biocompatible substance may be biodegradable. As used herein biodegradable means that a substance can chemically or enzymatically break down or degrade within the body. A biodegradable substance may form non-toxic components when it is broken down. Moreover, the biocompatible substance can be biologically neutral, meaning that it lacks specific binding properties or biorecognition properties.

Liposomes are artificial single, oligo- or multi-lamellar vesicles having an aqueous core and being formed from amphipathic molecules. The drug or diagnostic cargo may be trapped in the core of the liposome or disposed in the membrane layer or at the membrane surface. Today, liposomal vectors are the most important group of the non-viral delivery systems.

Because our carrier system consists of neutral or anionic coated liposomes which may contain therapeutics and/or diagnostic agents, following intravenous administration, such liposomal vehicles have been found to have a prolonged systemic circulation time. This prolonged circulation time is due to their small size and hydrophilic coating which may minimize uptake by the mononuclear phagocyte system and to their high molecular weight which prevents renal excretion. Liposome incorporated drugs may accumulate in tumours to a greater extent than the free drug and show reduced distribution into untargeted areas such as the heart. Accumulation of liposomes in malignant or inflamed tissues may be due to increased vascular permeability and impaired lymphatic drainage. The tumour vessels are more leaky and less permselective than normal vessels. Several *in vivo* studies have shown that liposomes are able to improve the efficiency of anticancer drugs against leukaemia and solid tumours.

PEG has many qualities that make it a desirable biocompatible ligand linked as part of the carrier of this invention. First, PEG is commercially available in a variety of molecular masses at low dispersity (Mw/Mn<1.1). It has been shown that PEG2000 will mask lipid-linked antibodies to a lesser degree than PEG5000 (MW of 5000 Dalton) (Mori A et al., Influence of the Steric Barrier Activity of Amphipathic Poly(ethyleneglycol) and Ganglioside GM1 on the Circulation Time of Liposomes and on the Target Binding of Immunoliposomes In Vivo, FEBS Lett. 284(2), 263-266, 1991). The studies indicated that PEG does not exhibit specific affinity for any organ and that its accumulation in tumour tissue is mainly governed by the level of hyperpermeable tumour vasculature (enhanced permeability retention (EPR) effect), irrespective of the molecular mass of the polymer and the tumour loading site. The EPR effect is considered as a passive targeting method, but drug targeting could be further increased by binding to ligands such as antibodies. Targeted liposomes can serve for the delivery of drug to tumours, inflamed tissues or endosomal compartments.

The carrier molecule may also include at least one lysis agent connected to the biodegradable cationic polymer coating. The lysis agent can be selected to break down a biological membrane such as a cell, endosomal, or nuclear membrane, thereby allowing the polyanionic macromolecule to be released into the cytoplasm or nucleus of the cell. As a result of the presence of the lysis agent, the membrane undergoes lysis, fusion, or both. Lysis agents also include viral peptides and synthetic compounds that can break down a biological membrane. A lytic peptide is a chemical grouping which penetrates a membrane such that the structural organization and integrity of the membrane is lost. As an example of a pH-sensitive endosomal lytic peptide is GLFEALLELLESLWELLLEA or GLFEALEELWEAK(e-G-dipalmitoyl) (MacLaughlin FC, Mumper RJ, Wang J, Tagliaferri JM, Gill I, Hinchcliffe M, Rolland AP. Chitosan and depolymerized chitosan oligomers as condensing carriers for in vivo plasmid delivery. J Cont Release 56:259-272, 1998).
The lysis agent may also be covalently linked to the cationic polymer coating by a linker. Such linkers can be 1-Ethyl-3-[3-dimethylaminopropyl] carbodiimide hydrochloride (EDAC) and *N*hydroxysuccinimide (NHS), a PEG fragment, a polypeptide, a linear polymer containing an ester bond or an amide bond or a disulfide bond. The linkers are preferably biodegradable linkers.

The carrier molecule may also include at least one targeting moiety connected to the biodegradable polycation coating. The targeting moiety can be selected to bind to a specific biological substance or site herein referred to as the receptor. Thus, the targeting moiety can be chosen based on its ability to bind to a receptor molecule expressed in a specific cell type or specific tissue allowing the polyanionic therapeutic agent to be selectively delivered to the cell or tissue. A targeting moiety refers to those moieties that bind to a specific biological substance or site. The biological substance or site is considered the target of the targeting moiety that binds to it. Ligands are one type of targeting moiety. Ligands have a selective (or specific) affinity for another substance known as the receptor. Because the ligand has a specific affinity for the receptor, the ligand binds to the receptor selectively over other molecules. This selective binding allows for the selective delivery of the polyanionic therapeutic to the target cell. Examples of ligands suitable for targeting cells are an antigen, a hapten, a vitamin, a protein, a polypeptide, biotin, nucleic acids, DNA, RNAi, aptamers, polynucleic acids, a polysaccharide, a carbohydrate, a lectin, a lipid and combination thereof, an antibody Fab or a fragment thereof.

The receptor for a ligand is an important consideration in selecting a ligand to target a cell. The receptor functions as a type of biorecognition molecule that selectively binds to the ligand. A receptor can be a protein such as an antibody or a non-protein binding body. As used herein an antibody refers to all classes of antibodies including monoclonal antibodies, chimeric antibodies, Fab fractions, and derivatives thereof.

The carrier of the present invention may also contain covalently nona-D-arginine with or without a spacer as indicated below to the polycation coating to improve target cell penetration.

The invention also provides a method of transporting a polyanionic molecule across the biological barriers of the cell. The cell can be a cell derived from an organism such as hepatocytes, liver cells, kidney cells, brain cells, bone marrow cells, nerve cells, heart cells, spleen cells, stem cells and co-cultures of the above. Moreover, the cells may be from established cell lines such as those available from the American Type Culture Collection (ATCC).

The method of delivering the polyanionic macromolecule to the cell includes complexing the polyanionic macromolecule to the carrier system of the present invention to create a complex. The carrier complex may enter the cell by endocytosis and then escape from the vesicles to gain access to the cytoplasm of the cell. If the target cell is within a cell culture *in vitro*, the cell can be contacted with the complexed carrier system. If the target cell is within an organism *in vivo*, the complex may be administered by injecting a solution containing the complex into the circulatory system of the organism. A carrier molecule with a targeting moiety attached will allow the complex to be directed to a target cell with a target corresponding to the targeting moiety. The polyanionic molecule/carrier complex may be administered to an organism by intramuscular, intra-peritoneal, intra-abdominal, subcutaneous, intravenous, and intra-arterial delivery. Other methods of administration of the complex include parenteral, topical, transdermal, transmucosal, inhaled, and insertion into a body cavity such as by ocular, vaginal, buccal, transurethral, rectal, nasal, oral, pulmonary and aural administration.

The polyanionic molecule can be selected from a number of macromolecules that are useful in the treatment of disease or in laboratory experimentation. In certain configurations of the complex, the polyanionic macromolecule is a nucleic acid such as RNAi, siRNA, DNA, or a combination or derivative thereof. The nucleic acid can be, for example, genomic DNA, plasmid DNA, synthetic DNA, or RNA. Other types of nucleic acids that can be used with the carrier molecule of present invention are, for example, an antisense oligonucleotide, ribozyme, DNAzyme, chimeric RNA/DNA oligonucleotide, phosphorothioate oligonucleotide, 2'-O-methyl oligonucleotides, DNA-PNA conjugates, DNA-morpholino-DNA conjugates, and combinations thereof.

### Example 1 : Delivery of RNAi (such as siRNA, shRNA, miRNA)

It has been discovered that polysaccharide nanocapsules having an antibody such as EGFR covalently attached on the surface can substantially increase the nanocapsule affinity for the target compared to the non targeted counterparts. The nanocapsules directed against the receptor can efficiently bind to and become internalized by cancer cells, resulting in targeted intracellular drug delivery. These targeted nanocapsules efficiently bind to and become internalized by cancer cells *in vitro*, resulting in targeted intracellular drug delivery of siRNA.

While the principle of antibody-conjugates to target cancer cells has been around for some time, in melanoma this strategy poses an additional problem due to the scarcity of suitable cell surface targets that are required for our specific system. Melanoma markers are generally comprised of 4 types (adapted from Medic S, Pearce RL, Heenan PJ and Ziman M. Molecular markers of circulating melanoma cells. Pigment Cell Res 20; 80-91, 2006):
1. Markers involved in melanogenesis. While most specific for the melanocytic lineage, TYR, MITF, PAX3, TRP-1, TRP-2, and gp100 are not expressed on the cell surface and cannot be targeted with antibodies.
2. Melanoma-associated antigens such as are MART-1/Melan-A, p97, GalNAc-T, MIA and MUC18/MCAM are fairly specific however they are either also expressed on many other normal tissues or they are T cell antigens.
3. Tumour-associated antigens are more highly expressed in melanoma, such as cell adhesion molecules, angiogenesis factors, MAGE-A3, S100b, YKL-40, CRP and CRT-MAA. However, these markers too are not specific enough for our purposes or not expressed on the cell surface.
4. Finally, there are markers associated with tumour cell growth, proliferation and migration. Examples are VEGFR, NF-kB, ATF-2, FOS, JUN, MK167, TOP2A, BIRC5, STK6. However, these factors are either not expressed on the cell surface, or not specific enough to be used to target melanoma.

However, there are two cell surface markers, EGFR and TROY, that we believe are excellent candidates for targeting by our antibody-coupled nanocapsules.

### 1. EGFR.

EGFR (ErbB-1) is member of the epidermal growth factor family that includes 3 other members (ErbB-2-4). EGFR is a type 1 receptor tyrosine kinase that is involved in processes related to cellular differentiation and proliferation. It has been well-established that its dysregulation, either via activating mutations or increased expression, contributes to several types of cancers (Woodburn JR. The epidermal growth factor receptor and its inhibition in cancer therapy. Pharmacol Ther 82:241- 50, 1999). Apart from epithelial cancers, EGFR is expressed in melanocytic lesions (Ellis DL, King LE, Nanney LB. Increased epidermal growth factor receptors in melanocytic lesions. J Am Acad Dermatol 27:539-46, 1992; Sparrow LE, Heenan PJ. Differential expression of epidermal growth factor receptor in melanocytic tumours demonstrated by immunohistochemistry and mRNA in situ hybridization. Australas J Dermatol, 40:19-24, 1999) and a correlation between EGFR expression and tumour progression was noted. Indeed, genetic studies showed amplification of the *EGFR* gene in a number of cases of melanoma. Consistent with these studies, amplification was more commonly observed in metastatic tumours than early-stage disease (Bastian BC, LeBoit PE, Hamm H, Brocker EB, Pinkel D. Chromosomal gains and losses in primary cutaneous melanomas detected by comparative genomic hybridization. Cancer Res 58:2170-5, 1998; Slovak ML, Persons D, Collins JM, Zhang F, Sosman JA, Tcheurekdjian L. Targeting multiple genetic aberrations in isolated tumour cells by spectral fluorescence in situ hybridization. Cancer Detect Prev 26:171-9, 2002; Udart M, Utikal J, Krahn GM, Peter RU. Chromosome 7 aneusomy: a marker for metastatic melanoma? Expression of epidermal growth factor receptor gene and chromosome 7 aneusomy in nevi, primary malignant melanomas and metastases. Neoplasia 3:245-54, 2001). Because of these findings, EGFR has become a popular clinical target. One therapeutic approach is the development of small molecules such as gefitinib (Iressa), which inhibit its kinase activity. The other strategy is by using monoclonal antibodies that interfere with ligand binding, such as cetuximab.

Even though clinical trials in melanoma with EGFR inhibitors have met with disappointment for reasons that are not yet fully understood (Sosman JA, Puzanov I. Molecular Targets in Melanoma from Angiogenesis to Apoptosis. Clin Cancer Res 12:2376s-2383s, 2006), EGFR expression in melanoma has been considered specific enough to be targeted with EGFR-targeting molecular tools in clinical trials. Based on these studies we decided to use an anti-EGFR antibody that is known to be internalized as one of our antibodies to be conjugated to our nanocapsules. As we will show in our preliminary studies, this strategy is highly promising for the EGFR-dependent delivery of both siRNA and chemotherapeutic drugs into melanoma cells.

### 2. TROY

Recently discovered TROY is a novel, highly specific melanoma-associated type I transmembrane receptor member of the TNF receptor superfamily (TNFRSF) that is aberrantly reexpressed in melanoma (Spanjaard RA, Whren KM, Graves C, Bhawan J. Tumour necrosis factor receptor superfamily member TROY is a novel melanoma biomarker and potential therapeutic target. Int J Cancer 120:1304-10, 2007). TNFRSF members comprise a very large family who, on a macroscopic scale, are important for organizing permanent multicellular structures such as lymphoid organs, hair follicles, sweat glands and bone but also transient structures and activities such as the lactating mammary gland and wound healing (Locksley RM, Killeen N, Lenardo MJ. The TNF and TNF receptor superfamilies: integrating mammalian biology. Cell 104:487-501, 2001). TNFRSF members are directly coupled to signaling pathways for cell proliferation and differentiation, and are well-studied with respect to their function in acute immune responses such as inflammation. The other major activity is induction of apoptosis (de Thonel A, Eriksson JE. Regulation of death receptors-Relevance in cancer therapies. Toxicol Appl Pharmacol 207(2 Suppl):123-32, 2005).
TROY is a relatively underexplored molecule, although some aspects have been described. During mouse embryogenesis, TROY is widely expressed (Eby MT, Jasmin A, Kumar A, Sharma K, Chaudhary PM. TAJ, a novel member of the tumour necrosis factor receptor family, activates the c- Jun N-terminal kinase pathway and mediates caspase-independent cell death. J Biol Chem 275:15336- 42, 2000; Hisaoka T, Morikawa Y, Kitamura T, Senba E. Expression of a member of tumour necrosis factor receptor superfamily, TROY, in the developing olfactory system. Glia 45:313-24, 2004; Kojima T, Morikawa Y, Copeland NG, Gilbert DJ, Jenkins NA, Senba E, Kitamura T. TROY, a newly identified member of the tumour necrosis factor receptor superfamily, exhibits a homology with Edar and is expressed in embryonic skin and hair follicles. J Biol Chem 275:20742-7, 2000; Ohazama A, Courtney JM, Tucker AS, Naito A, Tanaka S, Inoue J, Sharpe PT. Traf6 is essential for murine tooth cusp morphogenesis. Dev Dyn 229:131-5, 2004; Pispa J, Mikkola ML, Mustonen T, Thesleff I. Ectodysplasin, Edar and TNFRSF19 are expressed in complementary and overlapping patterns during mouse embryogenesis. Gene Expr Patterns 3:675-9, 2003), but it is particularly highlyexpressed in neuroepithelial cells where it may function to regulate cell proliferation or maintenance of the undifferentiated state (Hisaoka T, Morikawa Y, Kitamura T, Senba E. Expression of a member of tumour necrosis factor receptor superfamily, TROY, in the developing mouse brain. Brain Res Dev Brain Res 143:105-9, 2003). However, after birth expression becomes highly restricted to hair follicles and neuron-like cells in parts of the brain (Hisaoka T, Morikawa Y, Kitamura T, Senba E. Expression of a member of tumour necrosis factor receptor superfamily, TROY, in the developing olfactory system. Glia 45:313-24, 2004; Hu S, Tamada K, Ni J, Vincenz C, Chen L. Characterization of TNFRSF19, a novel member of the tumour necrosis factor receptor superfamily. Genomics 62:103- 7, 1999; Ohazama A, Courtney JM, Tucker AS, Naito A, Tanaka S, Inoue J, Sharpe PT. Traf6 is essential for murine tooth cusp morphogenesis. Dev Dyn 229:131-5, 2004; Park JB, Yiu G, Kaneko S, Wang J, Chang J, He XL, Garcia KC, He Z. A TNF receptor family member, TROY, is a coreceptor with Nogo receptor in mediating the inhibitory activity of myelin inhibitors. Neuron 45:345-51, 2005; Pispa J, Mikkola ML, Mustonen T, Thesleff I. Ectodysplasin, Edar and TNFRSF19 are expressed in complementary and overlapping patterns during mouse embryogenesis. Gene Expr Patterns 3:675-9, 2003; Shao Z, Browning JL, Lee X, Scott ML, Shulga-Morskaya S, Allaire N, Thill G, Levesque M, Sah D, McCoy JM, Murray B, Jung V, Pepinsky RB, Mi S. TAJ/TROY, an orphan TNF receptor family member, binds Nogo-66 receptor 1 and regulates axonal regeneration. Neuron 45:353-9, 2005) and perhaps prostate (Eby MT, Jasmin A, Kumar A, Sharma K, Chaudhary PM. TAJ, a novel member of the tumour necrosis factor receptor family, activates the c-Jun N-terminal kinase pathway and mediates caspase-independent cell death. J Biol Chem 275:15336-42, 2000).

The ligand for TROY remains to be established but does not appear to be a known TNFRSF activating ligand (Mandemakers WJ, Barres BA. Axon regeneration: it's getting crowded at the gates of TROY. Curr Biol 15:R302-5, 2005). Recently a function for TROY in normal brain was established when it was found that it is a novel Nogo-66 receptor coreceptor that mediates inhibition of axonal regeneration by myelin inhibitors in the central nervous system (Park JB, Yiu G, Kaneko S, Wang J, Chang J, He XL, Garcia KC, He Z. A TNF receptor family member, TROY, is a coreceptor with Nogo receptor in mediating the inhibitory activity of myelin inhibitors. Neuron 45:345-51, 2005; Shao Z, Browning JL, Lee X, Scott ML, Shulga-Morskaya S, Allaire N, Thill G, Levesque M, Sah D, McCoy JM, Murray B, Jung V, Pepinsky RB, Mi S. TAJ/TROY, an orphan TNF receptor family member, binds Nogo-66 receptor 1 and regulates axonal regeneration. Neuron 45:353-9, 2005).

Regardless of its function, TROY presents an exceptional melanoma-specific membrane protein that can be targeted by specific antibodies against its extracellular domain. It is likely that TROY is also internalized which may increase transfection efficiency, and siRNA and drug delivery to the tumour cell (Schütze S, Tchikov V, Schneider-Brachert W. Regulation of TNFR1 and CD95 signalling by receptor compartmentalization. Nat Rev Mol Cell Biol.9:655-62, 2008).

Selecting a therapeutic siRNA to inhibit melanoma cell proliferation.

The next issue is the selection of a suitable therapeutic siRNA to incorporate in our antibodycoupled nanocapsules that can effectively block melanoma cell proliferation. An excellent target for siRNA (as well as kinase inhibitors) is BRAF. There are 3 RAF genes (ARAF, BRAF and RAF1) that encode kinases that serve as down-stream effectors of RAS, but BRAF is particularly important for the development of melanoma. 70% of melanomas contain activating mutations in BRAF (Davies H, Bignell GR, Cox C, Stephens P, Edkins S, Clegg S, Teague J, Woffendin H, Garnett MJ, Bottomley W, Davis N, Dicks E, Ewing R, Floyd Y, Gray K, Hall S, Hawes R, Hughes J, Kosmidou V, Menzies A, Mould C, Parker A, Stevens C, Watt S, Hooper S, Wilson R, Jayatilake H, Gusterson BA, Cooper C, Shipley J, Hargrave D, Pritchard-Jones K, Maitland N, Chenevix-Trench G, Riggins GJ, Bigner DD, Palmieri G, Cossu A, Flanagan A, Nicholson A, Ho JW, Leung SY, Yuen ST, Weber BL, Seigler HF, Darrow TL, Paterson H, Marais R, Marshall CJ, Wooster R, Stratton MR, Futreal PA. Mutations of the BRAF gene in human cancer. Nature 417:949-54, 2002; Mercer KE, Pritchard CA. Raf proteins and cancer: BRAF is identified as a mutational target. Biochim Biophys Acta 1653:25-40, 2003) which are also present in premalignant atypical or dysplastic nevi (Yazdi AS, Palmedo G, Flaig MJ, Puchta U, Reckwerth A, Rütten A, Mentzel T, Hügel H, Hantschke M, Schmid-Wendtner MH, Kutzner H, Sander CA. Mutations of the BRAF gene in benign and malignant melanocytic lesions. J Invest Dermatol 121:1160-2, 2003). Almost 90% of BRAF mutations are of the V600E variety leading to constitutive kinase activation (Wan PT, Garnett MJ, Roe SM, Lee S, Niculescu-Duvaz D, Good VM, Jones CM, Marshall CJ, Springer CJ, Barford D, Marais R; Cancer Genome Project. Mechanism of activation of the RAF-ERK signaling pathway by oncogenic mutations of B-RAF. Cell 116:855-67, 2004; Wellbrock C, Karasarides M, Marais R. The RAF proteins take centre stage. Nat Rev Mol Cell Biol 5: 875-85, 2004) and uncontrolled stimulation of cell proliferation.

Another feature that makes BRAF attractive for our studies is that RNAi approaches have already shown that knock down of BRAF results in reduced tumour growth in both cellular and xenograft animal models (Hoeflich KP, Gray DC, Eby MT, Tien JY, Wong L, Bower J, Gogineni A, Zha J, Cole MJ, Stern HM, Murray LJ, Davis DP, Seshagiri S. Maintenance in Melanoma Models. Cancer Res 66: 999-1006, 2006). Note that validated siRNAs against BRAF are commercially available. Thus, siRNA directed against BRAF is our selected method.

### Nanocapsule preparation

Phospholipon 90 G (phosphatidyl Choline) was obtained from Lipoid (American Lecithin Co., New Jersey), Cholesterol was obtained from Barnet Inc. (Englewood Cliffs, NJ), Protosan UPB 80/20 (High Purity Chitosan, Molecular weight, 20,000 Dalton) was obtained from NovaMatrix (FMC Biopolymer, Philadelphia, PA). Protosan is a highly purified form of chitosan, characterized by a prevalence of amino groups on the □-D-glucose backbone which are available for covalent attachment to protein and peptide targeting agents. The normal mouse IgG and the EFGR (R-1) mouse monoclonal IgG2b were obtained from Santa Cruz Biotechnology, the EDAC, NHS, Ethanolamine Hydrochloride and FITC were obtained from Sigma-Aldrich (St Louis, MO).

Liposomes were prepared using the lipid-film method. Multilamellar liposomes (MLL), composed of high purity phosphatidylcholine (PC), and cholesterol (Chol) at a molar ratio of 3:1 (PC:Chol) were prepared by a lipid-film method (Szoka F and Paphadjopoulos D. Comparative Properties and methods of preparation of lipid vesicles (liposomes). Ann Rev Biophys Bioeng 9:467- 508, 1980). The lipid concentration in this initial suspension was 60 µmol/ml. To prepare the lipid film 3 grams of Phospholipon 90 G (Phosphatidyl Choline) and 0.5 grams of cholesterol were dissolved in 10 ml of chloroform. The solution was evaporated overnight and then vacuum evaporated for 1 hour to remove any chloroform residue remaining in the film. The lipid film was then rehydrated with 10 ml of PBS and sonicated for 15 minutes. The sonicated liposomes were then coated with the biopolymer to form nanocapsules as described (Takeuchi H, Yamamoto H, Niwa T, Hino T, Kawashima Y. Enteral absorption of insulin in rats from mucoadhesive chitosancoated liposomes. Pharm Res 13:896-901, 1996). A 0.5% solution of protosan was obtained by dissolving 0.5 grams of Protosan in 100 ml of water with 0.36 grams of lactic acid. 0.5 ml of protosan and 0.5 ml of liposomes were then stirred at room temperature for 1 hour.

### IgG and EGFR coupling solutions

The control IgG antibody solution was prepared by stirring 500 µl of 200 µg/0.5ml normal mouse polyclonal IgG (IgG-pAb), 200 µl of 400 mmol EDAC, and 200 µl of 100 mmol NHS at room temperature for 1 hour, according to a modification of the covalent EDAC/NHS amine crosslinking method (Endoh H., Suzuki Y. and Hashimoto Y. Antibody coating of liposomes with 1-Ethyl-3-(3-Dimethyl-Aminopropyl)Carbodiimide and the effect on target specificity. J Immun Meth 44:79-85, 1981). Similarly, the EGFR antibody solution was prepared by stirring 330 ml of 200 mg/ml EGFR (R-1):sc-101 mouse monoclonal IgG2b (EGFR-mAb), 200 µl of 400 mmol EDAC, and 200 ml of 100 mmol NHS at room temperature for 1 hour.

### IgG-pAb and EGFR-mAb nanocapsules

To prepare the control IgG-pAb nanocapsules we added 50 µl of the nanocapsules to the coupling IgG solution and stirred at room temperature for 150 minutes. We then added 10 µl of FITC (50 mg/ml) and incubated at room temperature for 1 hour with occasional shaking. We then added 20 µl of 1 M Ethanolamine HCl to the mixture. The excess FITC was removed by repetitive washing of the nanocapsules in PBS. The mixture was then split in 200 µl aliquots and frozen at - 20°C for 2 hours. The frozen aliquots were then lyophilized for 48 hours. Similarly, to prepare the EGFR-mAb nanocapsules we added 70 µl of the nanocapsules to the EGFR solution and stirred at room temperature for 150 minutes. We then added 10 ml of FITC and incubated at room temperature for 1 hour with occasional shaking. We then added 20 µl of 1 M Ethanolamine HCl to the mixture. The mixture was then split into 200 µl aliquots and frozen at -20°C for 2 hours. The frozen aliquots were then lyophilized for 48 hours.

### Complexation of validated siRNA

5 nM of 3'Alexa Fluor 488-labeled validated BRAF siRNA (QIAGEN Inc., Valencia, CA) was dissolved in 200 mg of DEPC-treated water, and then used to rehydrate the lyophilized mAbcoupled nanocapsules aliquots for 30 min at RT. The entrapment procedure was performed immediately before use.

### Cells and reagents

A375 human melanoma cells, which harbor an activated mutant form of BRAF (V600E), and have high EGFR expression, were obtained from the ATCC and cultured under standard conditions (Demary, K, Wong L, Spanjaard RA. Effects of retinoic acid and sodium butyrate on gene expression, histone acetylation and inhibition of proliferation of melanoma cells. Cancer Lett 163:103-7, 2001). DOX was obtained from Sigma (St. Louis, MO) and diluted to a 100 mg/ml stock solution just before addition to the nanocapsules.

### Transfections and fluorescence microscopy

A375 cells were seeded in 24 well-plates and grown until 50-70% confluent. Nanocapsules rehydrated in DEPC-treated water were added in different doses and left o/n. The next day, media was removed and replaced by fresh media, and transfection efficiency (# fluorescent cells/# total number of cells counted by bright field) was determined by fluorescence microscopy using an Olympus IX 51 inverted microscope with phase contrast, and fluorescence capabilities coupled to a digital imaging system. FITC was detected via a #41001 filter, Alexa Fluor 488 was analyzed via a #41002 filter (Chroma technology Corp., Rockingham, VT). Fluorescence was monitored and detected for up to 7 days after transfection for each experiment.

### Cell proliferation (MTS) assay

To assess the effects of DOX-loading of EGFR-coupled nanocapsules on growth of A375 cells, cells were seeded in quadruplicate in a 96-well plate at 7,500 cells/well in a volume of 100 µl/well. The next day, cells were treated with EGFR-mAb nanocapsules ± DOX at two different doses: 1.6 or 2.5 mg in 10 µl media for 1 or 3 hr resp. before media with particles was removed and again replaced by 100 µl regular media/well. After 2 days, viable cell numbers were determined by CellTiter 96 Aqueous One Solution Cell Proliferation Assay Kit (Promega, Madison, WI) which measures bioreduction of MTS into a soluble formazan in viable cells which can be determined in a microplate reader at 490 nM.

### Results

Two sets of experiments were performed with the IgG-pAb and EGFR-mAb nanocapsules. The lyophilized nanocapsules were rehydrated in 200 ml of DEPC treated water 30 minutes before use. They were added in different quantities to 1 ml wells containing A375 melanoma cells and incubated for approximately 24 hours before observation by fluorescence microscopy that allowed quantitative assessment of transfection efficiency.

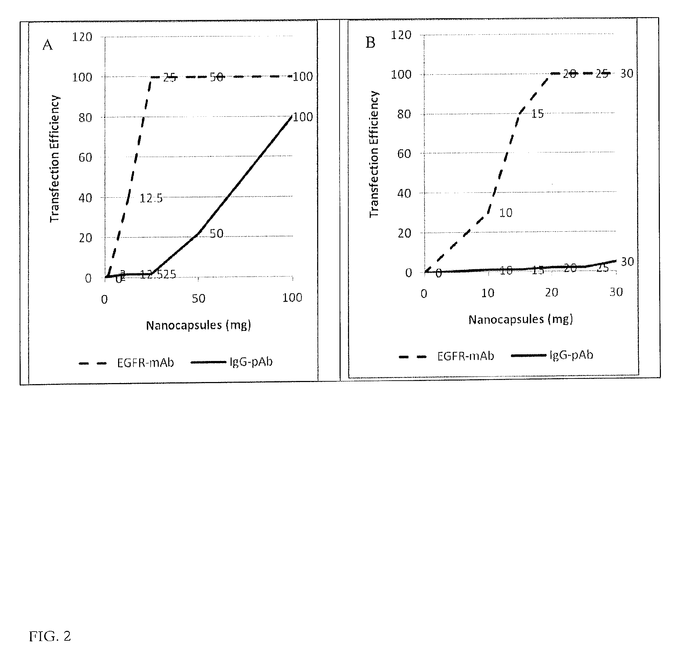

The results in Figure 2, which show the excellent reproducibility of our system, clearly demonstrate that the presence of the EGFR-mAb on the nanocapsule dramatically increases the transfection (delivery) efficiency of the nanocapsules compared to the IgG-mAb control nanocapsules. At 20 mg, 100% of cells are engaged by the EGFR-coupled nanocapsules whereas IgG control nanocapsules are essentially completely ineffective.
We estimate that the difference in transfection efficiency mediated by the EGFR-mAb is at least 10-fold. Thus, the coupling of a monoclonal, melanoma cell surface protein-targeting antibody to our nanocapsules greatly increases affinity for the intended cancer cell. These results imply that our system will be highly suitable for delivery of anticancer agents such as siRNA, and chemotherapeutic drugs. These questions were addressed in the following experiments.

First, we loaded FITC-labeled nanocapsules with a validated AlexaFluor 488-labeled siRNA directed against BRAF, and repeated the above described experiments.
As shown in Figure 3, at 20 mg, again 100% of A375 cells are transfected with the EGFR-antibody-coupled nanocapsules because the bright field and FITC-filter obtained images completely overlap. Interestingly, we find that the same overlap exists with the images detecting the BRAF siRNA. In contrast, no FITC derived fluorescence is detectable in the IgG-coupled control nanocapsules.
Thus, our EGFR-mAb nanocapsules appear to be an excellent, highly specific delivery system for the therapeutic BRAF siRNA.

The delivery of the carrier to the target cell is virtually fully ligand-dependent. In addition, in terms of stability of the complex between RNA and the cationic polymer, we could still detect RNA at least 7 days after transfection *in vitro*.

It also deserves mentioning that very little cell death was observed even at the highest doses after several days of treatment, although proliferation was mildly inhibited after prolonged incubation. The low-grade cytotoxicity of our nanocapsules, when combined with its longevity in tissue culture, which was confirmed on several other non-related epithelial cell types in these tissue culture experiments, is an extremely important characteristic for our nanocapsules being clinically suitable delivery agents. These aspects should be confirmed in *in vivo* studies.

### Example 2 : Delivery of chemotherapeutic agent

siRNA holds great promise as it allows specific functional knock-down of critical genes that drive tumour growth and/or survival. However, at the same time, these antibody-conjugated nanocapsules may also be exceptionally suited to deliver extreme localized (because to cancer cells only) chemotherapeutics. The most frequently given drug for advanced stage melanoma is Dacarbazine (DAC). Unlike other chemotherapeutics such as Doxorubicin (DOX), which is essentially ineffective, DAC has produced response rates in the 10-20% range and in rare cases complete remissions have been observed in melanoma patients. Generally, these responses do not result in increased survival and only provide temporary results (McLoughlin JM, Zager JS, Sondak VK, Berk LB.Treatment Options for Limited or Symptomatic Metastatic Melanoma. Cancer Control 15:239, 2008). It is conceivable that targeted delivery of chemotherapeutics with our nanocapsules is much more efficacious than systemic delivery.

Focusing further on our EGFR-mAb nanocapsules, as proof-of-concept experiment we next tested their ability to encapsulate the chemotherapeutic agent DOX, to which A375 melanoma cells are known to be sensitive. A375 cells were seeded in 96 well plates and treated with low and high dose nanocapsules ± DOX which are expected to correspond to 40 and 100% transfection efficiency, based on results shown in Figure 2. To minimize non intended effects due to leakage of DOX from the capsules into the media, cells were only incubated with the nanocapsules for 1 or 3 hr before they were removed and incubated in media. After 2 days, cell viability and proliferation was determined by MTS assay. As shown in Figure 4, absence of DOX has little effect regardless of incubation time. However, when loaded with DOX, a dose-and time-dependent inhibition of proliferation is observed. These results show that transfection of the melanoma cells by the nanocapsules *per se* does not inhibit proliferation, but that only the encapsulated anticancer agent affects the cancer cell's ability to proliferate. This again is an important characteristic for a therapeutic delivery agent. This should be confirmed in animal studies.

### Example 3 : Delivery of chemotherapeutic agent in vivo

### Immunoliposome preparation

Liposomes were prepared by a lipid film hydration-extrusion method as small unilamellar vesicles (approx. 100 nm in size) containing soy phosphatidylcholine, cholesterol and PEG-derivatized distearoyl-phosphatidylethanolamine containing a maleimide group for crosslinking to the ligand. Liposomes were prepared in 300 mM citrate buffer, pH 3.5, and outside buffer exchanged to HEPES-buffered saline at pH 7.5 for subsequent active loading of doxorubicin according to established procedures (Abraham et al., The Liposomal Formulation of Doxorubicin (DOX). Meth Enzym 391:71-97, 2005). Fab antibody fragments against TROY melanoma surface antigen were obtained by phage display screening of a mouse antibody gene library (Biosite Inc., San Diego, CA). To form immunoliposomes Fab thiol was conjugated to maleimide groups at the termini of PEG chains (PEG-Fab linkage) of the PEGylated vesicle forming lipid.

### In vivo therapeutic effect

Therapeutic effects of DOX-loaded immunoliposomes in nude mouse xenograft model. To test the *in vivo* therapeutic effects of the immunoliposomes, three different types were generated: 1) Control PEG liposomes without DOX, 2) same but loaded with DOX, and 3) immunoliposomes loaded with DOX. Next, 3 groups of 6 nude mice were injected in the flank with A375 melanoma cells, and after the tumour reached 50 mm3, mice received a single low-dose (20 mg/kg) injection in the tail vein with the nanocapsules. Tumour growth and body weight (as a marker for toxicity) was measured 3x/week. Mice were sacrificed when control tumours reached maximum allowable size. As shown in Fig. 5, the two non-targeted (no Fab) control groups did not show any tumour growth-inhibitory effects but the TROY-targeted immunoliposomes significantly blocked tumour growth. No differences in body weight were observed, indicative of low toxicity while systemic delivery of 20 mg DOX would have caused lethal toxicity within 8 days of treatment as known in the literature (eg. Tang et al. 2007). Thus, a single IV injection of low-dose DOX-loaded immunoliposomes showed significant therapeutic effects without associated toxicity. Note that the therapeutic effects are expected to be much stronger, and possibly curative with higher doses and/or frequency of injections.
These data strongly suggest that the immunoliposomes arrive at sufficient quantities at the tumour site to have a therapeutic effect while sparing normal tissues. This is a particularly exciting finding because if this mechanism is confirmed, conventional chemotherapeutic agents such as DOX that are clinically ineffective when given systemically, may be efficacious when delivered at high dose to the tumour site. This would be expected to have a dramatic impact on melanoma patient care.

## Claims

1. A carrier system, useful for administering therapeutic molecules to target cells, comprising ligand-targeted liposomes including on their surface a polymer chemically coupled to a ligand, said ligand
- possessing a high affinity for predefined receptor sites of the target cells;
- being capable of acting as a cell targeting agent of the target cells;
- being capable of acting as a cell internalization vector.

2. A system according to claim 1 comprising one or more of the features selected in the group consisting of
(a) the liposome is neutral or anionic and its vesicle-forming lipid is selected in the group consisting of hydrogenated soy phosphatidylcholine, cholesterol, DSPE-polyethyleneglycol-maleimide, distearoylphosphatidylcholine, sphingomyelin, diacyl glycerol, phosphatidyl ethanolamine, phosphatidylglycerol, distearylphosphatidylcholine and distearyl phosphatidylethanolamine ;
(b) the polymer is selected in the group consisting of a polysaccharide, polyglucosamine, oligoglucosamine, chitosan, a polypeptide, polyethylene glycol, polylysine, polyarginine and copolymers thereof ;
(c) the ligand is an antibody Fab or a fragment thereof.

3. A system according to claim 2 wherein the liposomes have a diameter ranging from 30 to 500 nanometers.

4. A system according to claims 2 or 3 wherein the liposome comprises at least one polyethyleneglycol vesicle-forming lipid having a maleimide group available for covalently attaching a ligand.

5. A system according to claims 2 to 4 wherein the vesicle-forming lipid has a molecular weight of less than or equal to 3400 Dalton.

6. A system according to claims 1 to 5 wherein the liposome comprises at least one distearyl phosphatidylethanolamine that is able to covalently attaching a polysaccharide.

7. A system according to claim 4 to 6 further including a spacer between the lipid-forming vesicle and the ligand; the spacer being selected in the list consisting of polysaccharide, polyethylene glycol, poly(alkylene glycol) of molecular weight less than 5000 Dalton.

8. A system according to claims 2 to 7 further comprising a ligand selected in the group consisting of an antigen, a hapten, a vitamin, a protein, a polypeptide, biotin, nucleic acids, DNA, RNA, aptamers, polynucleic acids, a polysaccharide, a carbohydrate, a lectin, a lipid and combination thereof and being
(i) connected to either the DSPE-polyethyleneglycol-maleimide lipid
- by using a coupling agent to convert the available amino groups into thiol groups for coupling to the maleimide group present on the pegylated lipid, or
- by first using tris(2-carboxyethyl)phosphine to reduce the available sulfide linkages on the ligand molecule into sulfidryl groups; or
(ii) connected to N-(3-Dimethylaminopropyl)-N'-ethylcarbodiimide hydrochloride to couple free available amino or carboxyl groups on the polysaccharide to free carboxyl or amino groups on the ligand.

9. A system according to claims 2 to 8 wherein the polymer (eg. polyethylene glycol, hyaluronic acid or chitosan) improves stealth and circulatory properties of the system.

10. A system according to claims 2 to 9 wherein the ligand is able to bind to a target cell or tissue specific antigen selected in the list consisting of prostate specific membrane antigen, TROY, lymphocyte antigens and tumour antigens.

11. A system according to claims 1 to 10 wherein the polymer contains an improving agent that is selected in the list consisting of the polyarginine D- and L-forms, nona-D-arginine, polylysine D- and L-forms, polyglucosamine and polyacetylglucosamine or mixtures thereof, and that is acting on the crossing, fusion or uptake of the carrier system across the target cell membrane.

12. A system according to claims 1 to 11 further comprising a lysis agent coupled to the polymer.

13. A system according to claim 12 wherein the lysis agent is a pH-sensitive endosomolytic peptide.

14. A system according to claim 8 wherein the second ligand is able to bind to a bacterial antigen, the extracellular domain of signalling membrane proteins selected in the list consisting of epidermal growth factor receptor, HER2/neu receptor, basic fibroblast growth factor receptor, vascular endothelial growth factor receptor, tumour necrosis factor receptor, insulin growth factor receptor, folate receptor, cell adhesion molecules proteins selected in the list consisting of E-selectin receptor, P-selectin receptor, L-selectin receptor, integrin receptors, chemokine receptors.

15. A system according to claims 1 to 14 useful for administering therapeutic molecules to internalizable target cells or antigen.
